# EUROPEAN PATENT SPECIFICATION

(11) **EP 2 738 165 B1**
(45) Date of publication and mention of the grant of the patent: **12.10.2016**
(21) Application number: 12195263.4
(22) Date of filing: 03.12.2012
(51) Int. Cl.: C07D 403/12

(54) **Method by means of phase transfer catalysis**
Verfahren mittels Phasentransferkatalyse
Procédé au moyen d'une catalyse par transfert de phase

(43) Date of publication of application: 04.06.2014
(73) Proprietor: WÖRWAG PHARMA GmbH & Co. KG, 71034 Böblingen (DE)
(72) Inventor: Staneva, Tinka, 2600 Dupnitsa (BG)
(74) Representative: Patentanwälte Ruff, Wilhelm, Beier, Dauster & Partner mbB

(56) References cited:
- EP-A1- 1 873 151
- WO-A2-2009/112954
- CZ-B6- 294 649
- US-A- 4 323 570

## Description

### Field of the invention and prior art

The invention relates to a new method for preparing a compound having the formula (I): 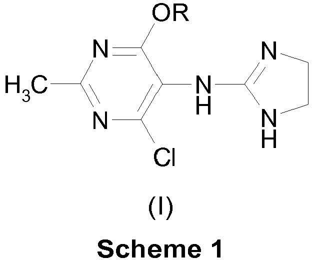 where R is an aliphatic and/or aromatic radical.

Moxonidine (4-chloro-N-(imidazolidin-2-ylidene)-6-methoxy-2-methyl-pyrimidin-5-amine) has the structural formula (Ia) as depicted in scheme 1 below and is used as an antihypertensive drug.

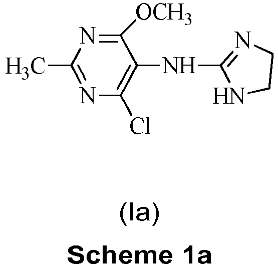

Moxonidine was approved for use in Germany in 1991 and is currently commercially available in Europe, e.g. in Germany, Austria and UK.

US 4,323,570 describes a method of preparing Moxonidine (Ia) by reacting 4,6-dichloro-2-methyl-5-(1-acetyl-2-imidazolin-2-yl)aminopyrimidine of formula (II) with about 2 equivalents of sodium methoxide in methanol under reflux, as depicted in Scheme 2.

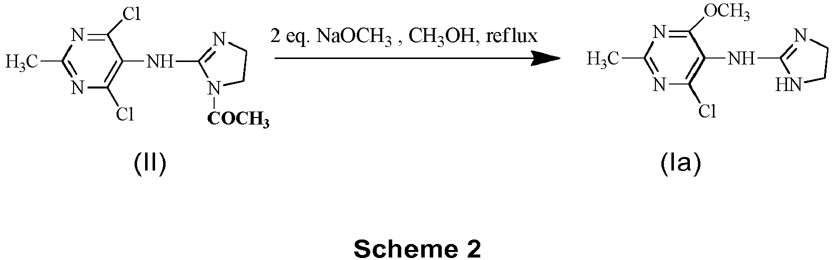

However, that preparation suffers from the principal withdrawal that boiling sodium methoxide is highly corrosive and toxic. Furthermore, according to US 4,323,570, Moxonidine may be obtained by crystallization from nitromethane which is also a toxic reagent.

CZ 294 649 B6 describes a method of preparing Moxonidine from 4,6-dichloro-2-methyl-5-(1-acetyl-2-imidazolin-2-yl)aminopyrimidine by methanolysis reaction using bimolar quantity of alkali metal carbonates, exempli gratia potassium carbonate at 47 °C to 52 °C or sodium bicarbonate at 65 °C.

EP 1 873 151 A1 describes an improved method of preparing Moxonidine with alkali metal hydroxides, carbonates and bicarbonates by using milder condition (ambient to reflux temperature and low molar excess of the base). On the one hand, alkali metal hydroxides, carbonates and bicarbonates are less corrosive and toxic. On the other hand, the reaction runs in suspension and additionally requires dissolving in acetic acid (in order to obtain a solution) and precipitation with ammonium hydroxide.

DE 29 37 023 A1 describes substituted 5-(2-imidazolin-2-yl)-aminopyrimidines of the general formula (III) as depicted in scheme 3 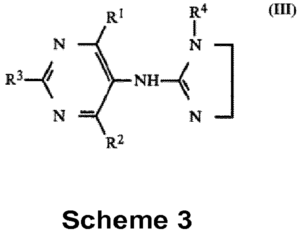 wherein R1, R2 and R3, which may be the same or different, represent a hydrogen or halogen atom, an alkoxy, alkylthio or alkyl group having 1 to 4 carbon atoms or a cycloalkyl group having 3 to 5 carbon atoms, wherein at least one of the substituents R¹, R² and R³ represents an alkylthio or a cycloalkyl group, and R⁴ represents a hydrogen atom or an aliphatic or aromatic acyl group, as well as their physiologically acceptable acid addition salts.

DE 28 49 537 A1 also describes substituted 5-(2-imidazolin-2-yl)-aminopyrimidines of the general formula (III) as depicted in scheme 3, wherein R¹, R² and R³, which may be the same or different, represent a hydrogen or halogen atom, an alkoxy or alkyl group having 1 to 4 carbon atoms and R⁴ represents a hydrogen atom or an aliphatic or aromatic acyl group as well as their physiologically acceptable acid addition salts.

WO 2009/112954 A2 discloses processes for the preparation of substantially pure ethylene glycol sulfonamide compounds.

### Object and solution

In view of the foregoing, the object underlying the present invention is to provide a method for preparing compounds corresponding to the formula (I), which in particular circumvents withdrawals known from the prior art using the example of Moxonidine.

This object is solved by a method having the features of independent claim 1. Preferred embodiments are reflected in dependent claims 2 to 15. The wording of all claims is herewith made to the contents of the description by explicit reference.

The present invention provides a method for preparing a compound having the formula (I): 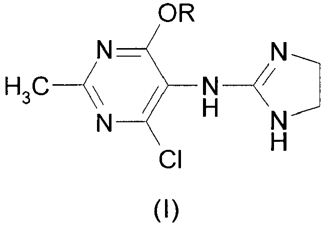 where R is an aliphatic and/or aromatic radical.

The method is especially featured in that 4,6-dichloro-2-methyl-5-(1-acetyl-2-imidazolin-2-yl)-aminopyrimidine and an alcohol are caused to react in the presence of a phase transfer catalyst.

In other words, reaction between 4,6-dichloro-2-methyl-5-(1-acetyl-2-imidazolin-2-yl)-aminopyrimidine and an alcohol is accomplished by means of a phase transfer catalyst, thereby yielding a compound having the formula (I).

As regards, preparing a compound having the formula (I), it surprisingly turned out that applying phase transfer catalysis requires significantly less harsh reaction conditions and additionally yields the desired compounds in high yields and high purities.

Phase transfer catalysis itself is a prominent technique for chemical synthesis. In that regard it is exemplarily referred to M. Makosza, B. Serafinowa, Rocz. Chem., 39, 1223 (1965); M. Makosza, W. Wawrzyewicz, Tetrahedron Lett., 4659 (1969); A. Brändström, K. Gustavii, Acta Chem. Scand., 23, 1215 (1969); C. M. Starks, J. Am. Soc., 93, 195 (1971); J. Jarrosse, C. R. Acad. Sci. Ser. C, 232, 1424 (1951)) and H. H. Freedman and R. A. Dubois, Tetrahedron Lett., 1975, 3251.

However, so far, to the best knowledge of the inventor, phase transfer catalysis has not been applied for the manufacture of a compound having the formula (I).

The inventor reproduced the examples 10 to 12 as described in EP 1 873 151 A1 and could confirm that preparation of Moxonidine ran in suspension in all the time of the reaction. Further, as mentioned at the outset, it could be confirmed that for the isolation of the Moxonidine it was necessary to add acetic acid to methanol medium to obtain a solution. However, under these circumstances, methanol could not be used again and had to be discard. Further, it turned out that for precipitation of Moxonidine, addition of ammonium hydroxide was necessary. Furthermore, if starting 4,6-dichloro-2-methyl-5-(1-acetyl-2-imidazolin-2-yl)-aminopyrimidine was coloured, the final product (Moxonidine) was coloured too. For decolourisation of Moxonidine, it was necessary to add activated carbon when everything was dissolved, to heat the solution for about a half an hour and then to filter. Furthermore, thus obtained Moxonidine contained the impurities 4,6-dimethoxy-2-methyl-5-(2-imidazolin-2-yl)-aminopyrimidine of formula (IV) and 4,6-dichloro-2-methyl-5-(2-imidazolin-2-yl)-aminopyrimidine of formula (V) as depicted in scheme 4 (and as described in reference examples 1, 2 and 3 of EP 1 873 151 A1 respectively) in an amount from 0.7 % to 1 %.

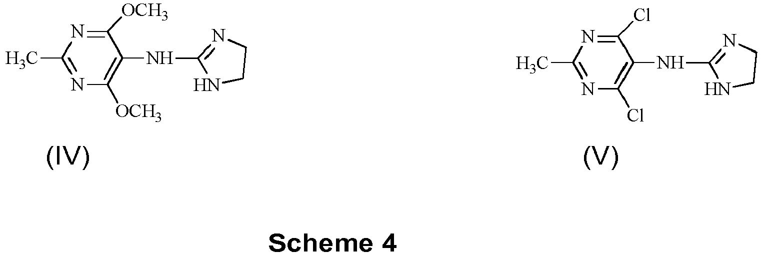

In contrast thereto, the present invention advantageously allows preparation of compounds having the formula (I) in a particularly economically viable, fast, very simple and environmentally benign manner while obtaining high yields, particularly more than 90 % and a very distinctive purity, particularly more than 99.5 %.

Without wishing to be bound by any theory, the mechanism underlying the method according to the present invention is based on that via the cation of the phase transfer catalyst alcoholat ions are transferred from an aqueous phase into an organic phase. The alcoholat ions then substitute the chlorine atoms at position 6 of the pyrimidine ring of 4,6-dichloro-2-methyl-5-(1-acetyl-2-imidazolin-2-yl)-aminopyrimidine on the basis of a nucleophilic substitution, thereby yielding compounds having the formula (I).

In a preferred embodiment, the alcohol is used in combination with at least a further organic solvent being not soluble or miscible in water. In other words, the reaction is preferably conducted using a solvent mixture of the alcohol and at least a further organic solvent which is not soluble or miscible in water.

The at least one further organic solvent may be selected from the group consisting of methylene chloride, chloroform, benzene, diethyl ether, diisopropyl ether, petroleum ether and mixtures thereof.

In a preferred embodiment, the alcohol is used in combination with methylene chloride.

The substituent R of formula (I) may be selected from the group consisting of a linear (i.e. unbranched) aliphatic radical, a branched aliphatic radical, a cyclic aliphatic radical and a halogenated aliphatic radical.

The aliphatic radical may be further selected from the group consisting of alkyl radicals, alkenyl radicals and alkinyl radicals.

Preferably, R is an alkyl radical. For example, R may be selected from the group consisting of methyl radical, ethyl radical, n-propyl radical, 2-propyl radical, n-butyl radical, tert. butyl radical, n-pentyl radical, n-hexyl radical, cyclohexyl radical, allyl radical, 3-buten radical, 2-propyn radical, 3-butyn radical, 2-butyn radical, 4-pentyn radical and combinations thereof.

According to an especially preferred embodiment, R is a methyl radical.

In combination or alternatively, R may be selected from the group consisting of a linear (i.e. unbranched) aromatic radical, in particular a linear alkyl aromatic radical, a branched aromatic radical, in particular a branched alkyl aromatic radical, a cyclic aromatic radical, a nitrated aromatic radical, a halogenated aromatic radical and combinations thereof.

Further, R may be a radical having aliphatic and aromatic moieties.

In accordance with the embodiments described in the preceding paragraphs, the alcohol used for the reaction may be selected from the group consisting of a linear (i.e. unbranched) aliphatic alcohol, a branched aliphatic alcohol, a cyclic aliphatic alcohol and a halogenated aliphatic alcohol.

The aliphatic alcohol may be further selected from the group consisting of alkanol, alkenol and alkinol.

Preferably, the alcohol is selected from the group consisting of methanol, ethanol, n-propanol, 2-propanol, n-butanol, tert. butanol, n-pentanol, n-hexanol, cyclohexanol, allyl alcohol, 3-buten-1-ol, 2-propyn-1-ol, 3-butyn-1-ol, 2-butyn-1-ol, 4-pentyn-1-ol and combinations thereof. Methanol is especially preferred.

In combination or alternatively, the alcohol may be selected from the group consisting of a linear (i.e. unbranched) aromatic alcohol, in particular a linear alkyl aromatic alcohol, a branched aromatic alcohol, in particular a branched alkyl aromatic alcohol, a cyclic aromatic alcohol, a nitrated aromatic alcohol, a halogenated aromatic alcohol and combinations thereof.

Further, the alcohol may have aliphatic and aromatic moieties.

In a preferred embodiment, the compound having the formula (I) is a 4-chloro-N-(imidazolidin-2-ylidene)-6-alkoxy-2-methyl-pyrimidin-5-amine, in particular selected from the group consisting of 4-chloro-N-(imidazolidin-2-ylidene)-6-methoxy-2-methyl-pyrimidin-5-amine, 4-chloro-N-(imidazolidin-2-ylidene)-6-ethoxy-2-methyl-pyrimidin-5-amine, 4-chloro-N-(imidazolidin-2-ylidene)-n-propoxy-2-methyl-pyrimidin-5-amine, 4-chloro-N-(imidazolidin-2-ylidene)-6-isopropoxy-2-methyl-pyrimidin-5-amine, 4-chloro-N-(imidazolidin-2-ylidene)-6-butoxy-2-methyl-pyrimidin-5-amine, 4-chloro-N-(imidazolidin-2-ylidene)-6-pentoxy-2-methyl-pyrimidin-5-amine, 4-chloro-N-(imidazolidin-2-ylidene)-6-hexoxy-2-methyl-pyrimidin-5-amine and 4-chloro-N-(imidazolidin-2-ylidene)-6-cyclohexoxy-2-methyl-pyrimidin-5-amine.

In an especially preferred embodiment, the compound to be prepared is Moxonidine (4-chloro-N-(imidazolidin-2-ylidene)-6-methoxy-2-methyl-pyrimidin-5-amine), i.e. R of formula (I) is a methyl radical, and the alcohol is methanol which is caused to react with 4,6-dichloro-2-methyl-5-(1-acetyl-2-imidazolin-2-yl)-aminopyrimidine.

The phase transfer catalyst is preferably selected from the group consisting of quaternary ammonium salt, phosphonium salt, crown ether, aza crown ether, cryptand, acyclic molecule being analogous to crown ether, for example oligoethylene glycol and/or polyethylene glycol, and mixtures thereof.

More specifically, the phase transfer catalyst may be selected from the group consisting of alkylammonium salt, alkylarylammonium salt and mixtures thereof, particularly from the group consisting of tetraalkylammonium salt, trialkylarylammonium salt such as trialkylbenzylammonium salt and mixtures thereof.

In a further or alternative embodiment, the phase transfer catalyst may be a quaternary ammonium salt which is selected from the group consisting of tetramethylammonium chloride, tetramethylammonium bromide, tetramethylammonium hydroxide, tetramethylammonium nitrate, tetramethylammonium perchlorate, tetramethylammonium hexafluorphosphate, tetramethylammonium tetrafluoroborate, tetraethylammonium chloride, tetraethylammonium bromide, tetraethylammonium hydroxide, tetraethylammonium nitrate, tetraethylammonium perchlorate, tetraethylammonium tetrafluoroborate, tetraethylammonium p-toluensulfonate, triethylmethylammonium chloride, trimethylhexylammonium bromide, trimethylpentylammonium chloride, trimethylpentylammonium bromide, trimethylpentylammonium iodide, trimethylpentylammonium methosulfate, trimethylbenzylammonium chloride, trimethylbenzylammonium bromide, trimethylbenzylammonium iodide, trimethylbenzylammonium hydroxide, trimethylbenzylammonium nitrate, trimethylbenzylammonium hexafluorophosphate, trimethylbutylammonium bromide, trimethyloctylammonium bromide, trimethydecylammonium bromide, triethylbenzylammonium chloride, triethylbenzylammonium bromide, triethylbenzylammonium hydroxide, triethylbenzylammonium tetrafluoroborate, trimethyldodecylammonium chloride, trimethyldodecylammonium bromide, trimethyltetradecylammonium chloride, trimethyltetradecylammonium bromide, trimethylhexadecylammonium bromide, dimethyldi(n-decyl)ammonium bromide, dimethyl-distearylammonium chloride, dimethylhexadecylethylammonium bromide, tri(-n-propyl)benzylammonium chloride, tetra-n-propylammonium chloride, tetra-n-propylammonium bromide, tetra-n-propylammonium iodide, tetra-n-propylammonium hydroxide, tetra-n-propylammonium hydrogensulfate, tri-n-butylmethylammonium chloride, tri-n-butylmethylammonium bromide, tetra-n-butylammonium chloride, tetra-n-butylammonium bromide, tetra-n-butylammonium iodide, tetra-n-butylammonium hydroxide, tetra-n-butylammonium nitrate, tetra-n-butylammonium perchlorate, tetra-n-butylammonium acetate, tetra-n-butylammonium phosphate, tetra-n-butylammonium hexafluorophosphate, tetra-n-butylammonium hydrogen sulphate, tetra-n-butylammonium sulphate, tetra-n-butylammonium trifluoromethanesulfonate, tri-n-butylbenzylammonium chloride, tri-n-butylbenzylammonium bromide, tri-n-butylbenzylammonium iodide, tetra-n-pentylammonium iodide, tetra-n-hexylammonium bromide, tetra-n-hexylammonium iodide, tetra-n-hexylammonium perchlorate, tetra-n-heptylammonium bromide, tetra-n-heptylammonium iodide, tetra-n-octylammonium bromide, tetra-n-dodecylammonium iodide, tetra-n-octadecylammonium iodide, tri-n-octylmethylammonium chloride (such as commercially available under the name Aliquat 336), tri-n-nonylmethylammonium chloride, tri-n-decylmethylammonium chloride, (1-hexadecyl) pyridinium chloride monohydrate, (1-hexadecyl) pyridinium bromide monohydrate and mixtures thereof.

In a further or alternative embodiment, the phase transfer catalyst may be a phosphonium salt selected from the group consisting of bis(triphenylphosphoranylyden)ammonium chloride, (1-hexadecyl)tri-n-butylphosphonium bromide, tetra-n-butylphosphonium bromide, tetraphenylphosphonium chloride, tetraphenylphosphonium bromide, tetraphenylphosphonium iodide, tetraphenylphosphonium tetrafluoroborate, tetraphenylphosphonium hexafluoroantimonate, (triphenylmethyl)triphenylphosphonium chloride and mixtures thereof.

An example for a suitable mixture of quaternary ammonium salts is a mixture of tri-n-octylmethylammonium chloride, tri-n-nonylmethyl-ammonium chloride and tri-n-decylmethylammonium chloride. Such a mixture is commercially available under the name Adogen 464.

In a further or alternative embodiment, the phase transfer catalyst may be a crown ether selected from the group consisting of benzo-15-crown-5, benzo-18-crown-6, 12-crown-4, 15-crown-5, 18-crown-6, cyclohexano-15-crown-5, dibenzo-18-crown-6, 4',4",(5")-di-tert-butyldibenzo-18-crown-6, dicyclohexano-18-crown-6, 2-hydroxymethyl-12-crown-4, 2-hydroxymethyl-15-crown-5, 2-hydroxymethyl-18-crown-6, 4-nitrobenzo-15-crown-5, 4-nitrobenzo-18-crown-6 and mixtures thereof.

In a further or alternative embodiment, the phase transfer catalyst may be an aza crown ether selected from the group consisting of 1,4,7,10-tetraazacyclododecane tetrahydrochloride (cyclen tetrahydrochloride), 1,4,8,11-tetraazacyclotetradecane (cyclam), 1,4,8,11-tetramethyl-1,4,8,11-tetraazacyclotetradecane, 1,7,10,16-tetraoxa-4,13-diazacyclo-octadecane, 1,4,10,13-tetrathia-7,16-diazacyclooctane and mixtures thereof.

In a further or alternative embodiment, the phase transfer catalyst may be an oligoethylene glycol and/or a polyethylene glycol selected from the group consisting of pentaethylene glycol, hexaethylene glycol, polyethylene glycol, in particular polyethylene glycol 200, polyethylene glycol 400, polyethylene glycol 600 and/or polyethylene glycol 1000, tris(3,6-dioxaheptyl)amine (TDA-1) and mixtures thereof.

Preferably, the phase transfer catalyst is used in an amount of from 0.05 mol % to 5.0 mol %, in particular 0.3 mol % to 3.0 mol %, preferably 0.1 mol % to 1.0 mol %, more preferably 0.1 mol %, per one mole 4,6-dichloro-2-methyl-5-(1-acetyl-2-imidazolin-2-yl)-aminopyrimidine.

In a further embodiment, 4,6-dichloro-2-methyl-5-(1-acetyl-2-imidazolin-2-yl)-aminopyrimidine is dissolved in a solvent mixture of the alcohol and preferably at least one further organic solvent being not soluble in water to yield an organic solution. With regard to the at least one further organic solvent, it is referred to the previous description.

The 4,6-dichloro-2-methyl-5-(1-acetyl-2-imidazolin-2-yl)-aminopyrimidine may be used for example with a purity of 87 % (without further purification before using it).

In a further embodiment, a mixture of the alcohol and preferably the at least one further organic solvent (being insoluble in water) is used in an amount of 120 ml per 5 g 4,6-dichloro-2-methyl-5-(1-acetyl-2-imidazolin2-yl)-aminopyrimidine.

The phase transfer catalyst is preferably added to the organic solution as mentioned in the preceding embodiments.

However, it may also be within the scope of the present invention to provide an organic solution which - from the very first - contains the phase transfer catalyst, i.e. to provide an organic solution containing from the very first a phase transfer catalyst along 4,6-dichloro-2-methyl-5-(1-acetyl-2-imidazolin-2-yl)-aminopyrimidine, the alcohol and preferably the at least one further organic solvent which is not soluble in water.

Alternatively, it may further be within the scope of the invention to add the phase transfer catalyst to an aqueous solution which will be more detailed in the following.

In a further embodiment, the reaction between the 4,6-dichloro-2-methyl-5-(1-acetyl-2-imidazolin-2-yl)-aminopyrimidine and the alcohol is caused in the presence of an inorganic base.

Preferably, the inorganic base is a metal hydroxide, in particular selected from the group consisting of alkali metal hydroxide, alkaline earth metal hydroxide and mixtures thereof.

More specifically, the inorganic base may be selected from the group consisting of lithium hydroxide, sodium hydroxide, potassium hydroxide, cesium hydroxide, barium hydroxide, magnesium hydroxide, calcium hydroxide, strontium hydroxide and mixtures thereof.

Preferably, the inorganic base is provided in form of an aqueous solution (i.e. an aqueous solution containing the inorganic base).

The aqueous solution may contain the inorganic base in an amount of from 10 % by weight to 52 % by weight, in particular 20 % by weight to 51 % by weight, preferably 40 % by weight to 50 % by weight, more preferably 50 % by weight, related to the total weight of the aqueous solution.

In a further embodiment, the aqueous solution is added to an organic solution as mentioned in the preceding embodiments, wherein the organic solution preferably additionally contains the phase transfer catalyst, thereby forming a two-phase-system of an organic phase and an aqueous phase.

In an expedient embodiment, the reaction occurs in solution. This advantageously accelerates the reaction rate.

In a further embodiment, heat is applied for preparing the compounds having the formula (I). For example, the reaction may be conducted at a temperature range of from 10 °C to 50 °C, in particular 18 °C to 40 °C, preferably 30 °C to 35 °C.

In a further embodiment, the 4,6-dichloro-2-methyl-5-(1-acetyl-2-imidazolin-2-yl)-aminopyrimidine and the alcohol are caused to react during 0.5 h to 5 h, in particular 1 h to 4 h, preferably 2 h to 3 h.

Further, the 4,6-dichloro-2-methyl-5-(1-acetyl-2-imidazolin-2-yl)-aminopyrimidine and the alcohol may be caused to react until complete consumption of 4,6-dichloro-2-methyl-5-(1-acetyl-2-imidazolin-2-yl)aminopyrimidine and/or of undesired by-products and intermediates, respectively such as 4,6-dichloro-2-methyl-5-(2-imidazolin-2-yl)-aminopyrimidine (see formula (V)).

To this means, the progress of the reaction may be monitored, in particular by means of high-pressure liquid chromatography (HPLC) and/or thin layer chromatography (TLC).

In order to stop the reaction, preferably after complete conversion into the compound having the formula (I), the aqueous solution and the organic solution, in particular being present as two distinct phases, namely as an organic phase and an aqueous phase, of a two-phase-system, are separated.

After separation, the aqueous solution and phase, respectively may be advantageously reused, whereas the organic solvent(s), i.e. the alcohol and preferably the at least one further water-insoluble organic solvent, are preferably removed, in particular evaporated, thereby yielding a residue of the compound having the formula (I) as crude product.

Thus, for example, the amount of a mixture of the alcohol and the at least one further water-insoluble organic solvent may be reduced to 25%, related to the solvent amount originally applied.

While the separated organic solvents may be advantageously also reused, the residue is typically subjected to further purification and isolation steps.

Preferably, the residue is suspended, thereby yielding a precipitate. This may be achieved by adding water or an aqueous liquid. Preferably more water is added than the expected amount of purified compound having the formula (I). For example, water is added in an excess of 10 o 15 divisible parts.

Subsequently, the precipitate is collected by means of filtration.

Thereafter, the precipitate may be washed with water or an aqueous liquid, preferably until the pH of the filtrate is about 7.

Thereafter, the precipitate is typically dried, for example by means of vacuum drying, thereby furnishing purified and in particular crystalline compound having the formula (I).

According to an especially preferred embodiment, the method according to the present invention comprises the following steps:
a) providing an organic solution containing 4,6-dichloro-2-methyl-5-(1-acetyl-2-imidazolin-2-yl)-aminopyrimidine and a solvent mixture of an alcohol and at least one further organic solvent which is insoluble in water,
b) adding a phase transfer catalyst to the organic solution,
c) adding an aqueous solution containing an inorganic base, preferably a metal hydroxide, to the organic solution including the phase transfer catalyst, thereby forming a two-phase system of an organic phase and an aqueous phase,
d) optionally heating the two-phase system,
e) separating the organic phase and aqueous phase,
f) removing, in particular evaporating, the organic solvents from the organic phase, thereby yielding a residue of crude compound having the formula (I),
g) suspending the residue, typically in water or in an aqueous liquid, resulting in a precipitate,
h) collecting the precipitate by means of filtration,
i) optionally washing the collected precipitate, preferably with water or an aqueous liquid, and
j) drying the washed precipitate, thereby yielding purified and in particular crystalline compound having the formula (I).

Having regard to further features and advantages in respect of the embodiment as described in the preceding paragraph, reference is made in its entirety to the whole description.

Finally, the advantages of the present invention shall be again summarized as follows:
The method according to the present invention represents a fast, very convenient and gentle approach including a very simple work-up (without the necessity to attend to laborious such as anhydrous conditions) for preparing compounds having the formula (I), preferably in a crystalline and in particular colourless form, with high yield and purity, in particular independently from whether 4,6-dichloro-2-methyl-5-(1-acetyl-2-imidazolin-2-yl)-aminopyrimidine is coloured or not.

Preferably, the method according to the present invention furnishes, in particular without additional purification, compounds having the formula (I) in purities above 95%, in particular above 98%, preferably above 99%, more preferably above 99.5 %.

Further features and advantages of the invention will be become clear from the following description of preferred embodiments in form of examples in conjunction with the subject matter of the dependent claims. The individual features can be realized either singularly or severally in combination in one embodiment of the invention. The preferred embodiments merely serve for illustration and better understanding of the invention and are not to be understood as in any way limiting the invention.

### Examples

HPLC measurements of Moxonidine samples were performed using HPLC system, equipped with Lichrospher 60 RP-select B, 5 µm, 250 mm x 4 mm, column, and a UV detector operated on 230 nm. Analyses were performed using mobile phase consisting 88 % buffer solution of pentanesulphonic acid sodium salt with pH = 3.5, adjusted with dilute sulphuric acid (H₂SO₄) and 12 % of acetonitrile.

In the following, 4,6-dichloro-2-methyl-5-(1-acetyl-2-imidazolin-2-yl)-aminopyrimidine is abbreviated as DMAIA.

### Example 1 (reference) - Preparation of Moxonidine by reaction of DMAIA with1 molar equivalent of potassium carbonate at 65 °C

**Potassium carbonate (2.4 g, 0.0174 mol, 1 molar equivalents) was add**ed to a suspension of DMAIA (5.0 g, 0.0174 mol) in methanol (40 ml) and the mixture was heated at 65 °C for 3 hours. Then the reaction mixture was cooled to ambient temperature and acetic acid (4 ml) and water (35 ml) were added. After stirring for half an hour, the reaction mixture was concentrated to about 1/4 of the volume. 25% solution of ammonium hydroxide (4 ml) was added and the mixture was stirred for one hour. A precipitate was collected by filtration, washed with water and dried at 50 °C to give 3.5 g of crude Moxonidine in 83.45 % yield and in 94.84% purity.

### Example 2 - Preparation of Moxonidine by phase transfer catalysis

Sodium hydroxide (10 ml, 50 % aqueous solution, 5.0 g, 0.125 mol) was added to a solution of DMAIA (5.0 g, 0.0174 mol, 87 % purity) in methanol (20 ml), methylene chloride (100 ml) and tetra-n-butylammonium bromide (0.32 g, 0.1 mol %). The reaction was slightly exothermic and for a quarter of an hour the temperature reached 30 °C. Then the solution was heated at 35 °C for 2 hours. The aqueous phase was separated by funnel and the solvents from the organic phase were distillated. The residue was suspended in water (50 ml) and the precipitate was collected by filtration, washed with water till pH ∼7 of the washing water and dried at 50 °C. The yield of Moxonidine was 3.43 g (93.97%; toward 87% of DMAIA purity) with a purity of 99.91 %.

### Examples 3-16

The same reaction, which is provided in example 2, was carried out using different catalysts and different conditions as described in tables 1 to 4.

**Table 1 - Preparation of Moxonidine by reaction in different concentration of sodium hydroxide in aqueous phase**

| No | DMAIA [g] ([mol]) | solv. ([ml]) | NaOH [g/ml], % H₂O | T [°C] | t [h] | crude yield [g] ([%]) | purity [%] | IV [%] | V [%] |
|---|---|---|---|---|---|---|---|---|---|
| 3 | 4.35 (0.0151) | MeOH (20) CH₂Cl₂ (100) | 5/10, 50 | 35 | 1.0 | 3.43 (94.02) | 99.91 | - | 0.0 6 |
| 4 | 4.35 (0.0151) | MeOH (20) CH₂Cl₂ | 4/10, 40 | 35 | 1.5 | 3.40 (93.20) | 99.73 | - | 0.1 2 |
| | | (100) | | | | | | | |
| 5 | 4.35 (0.0151) | MeOH (20) CH₂Cl₂ (100) | 3/10, 30 | 35 | 2.0 | 3.35 (91.83) | 99.86 | - | 0.0 6 |
| 6 | 4.35 (0.0151) | MeOH (20) CH₂Cl₂ (100) | 2/10, 20 | 35 | 6.0 | 2.88 (78.95) | 99.72 | - | 0.1 0 |

**Table 2 - Preparation of Moxonidine by reaction with different amount of catalyst**

| No | DMAIA [g] ([mol]) | solv. ([ml]) | Catalyst ([mol %]) | T. [°C] | t [h] | crude yield [g] ([%]) | purity [%] | IV [%] | V [%] |
|---|---|---|---|---|---|---|---|---|---|
| 7 | 4.35 (0.0151) | MeOH (20) CH₂Cl₂ (100) | TBABr (0.075) | 35 | 2.5 | 3.50 (95.94) | 99.75 | - | 0.06 |
| 8 | 4.35 (0.0151) | MeOH (20) CH₂Cl₂ (100) | TBABr (0.005) | 35 | 3.0 | 3.23 (88.54) | 99.51 | 0.0 1 | 0.10 |

**Table 3 - Preparation of Moxonidine by reaction without heating with 0.1 mol % of tetra-n-butyl ammonium bromide and different concentration of sodium hydroxide**

| No | DMAIA [g] ([mol]) | solv. ([ml]) | NaOH [g/ml], % H₂O | T. [°C] | t [h] | crude yield [g] ([%]) | purity [%] | IV % | V % |
|---|---|---|---|---|---|---|---|---|---|
| 9 | 4.35 (0.0151) | MeOH (20) CH₂Cl₂ (100) | 5/10, 50 | 30 | 1.0 | 3.50 (95.94) | 99.42 | 0.16 | 0.0 5 |
| 10 | 4.35 (0.0151) | MeOH (20) CH₂Cl₂ (100) | 4/10, 40 | 30 | 2.5 | 3.44 (94.30) | 99.52 | 0.04 | 0.0 6 |
| 11 | 4.35 (0.0151) | MeOH (20) CH₂Cl₂ (100) | 3/10, 30 | 30 | 6.0 | 3.36 (92.10) | 99.72 | - | 0.1 2 |

**Table 4- Preparation of Moxonidine by reaction with different catalysts in amount of 0.1 mol %**

| No | DMAIA [g] ([mol]) | solv. ([ml]) | Catalyst ([mol %]) | T. [°C] | t [h] | crude yield [g] ([%]) | purity [%] | IV % | V % |
|---|---|---|---|---|---|---|---|---|---|
| 12 | 4.35 (0.0151) | MeOH (20) CH₂Cl₂ (100) | TBAJ (0.1) | 35 | 3 | 3.51 (96.21) | 99.70 | 0.0 7 | 0.09 |
| 13 | 4.35 (0.0151) | MeOH (20) CH₂Cl₂ (100) | TEBACI (0.1) | 35 | 2 | 3.44 (94.30) | 99.66 | 0.0 2 | 0.14 |
| 14 | 4.35 (0.0151) | MeOH (20) CH₂Cl₂ (100) | TEBABr (0.1) | 35 | 2 | 3.47 (95.12) | 99.69 | 0.0 8 | 0.10 |
| 15 | 4.35 (0.0151) | MeOH (20) CH₂Cl₂ (100) | MTOACl (0.1) | 35 | 3.5 | 3.30 (90.46) | 99.72 | 0.0 4 | 0.11 |
| 16 | 4.35 (0.0151) | MeOH (20) CH₂Cl₂ (100) | TBAHS (0.1) | 35 | 2.5 | 3.44 (94.30) | 99.73 | - | 0.13 |

The separated aqueous phase can be used second time which reduce at the half the consumption of catalyst and sodium hydroxide.

The separated aqueous phase after the end of the reaction can be used for second time without new amounts of sodium hydroxide, water and catalyst.

### Example 17 - Preparation of Moxonidine by phase transfer catalysis by using aqueous phase second time

The separated aqueous phase was added to the solution of DMAIA (5.0 g, 0.0174 mol, 87% purity) in methanol (20 ml) and methylene chloride (100 ml). The reaction was slightly exothermic and for a quarter of an hour the temperature reached 30 °C. Then the solution was heated at 35 °C for 6 hours. The aqueous phase was separated by funnel and the solvents from the organic phase were distillated. The residue was suspended in water (50 ml) and the precipitate was collected by filtration, washed with water till pH ∼7 and dried at 50 °C. The yield of crude Moxonidine was 3.47 g (95.12%; toward 87% of DMAIA purity).

The mixture of methylene chloride and methanol can be reduced 25 %.

### Example 18 - Preparation of Moxonidine by phase transfer catalysis by using 25 % lower amount of solvents

Sodium hydroxide (10 ml, 50 % aqueous solution, 5.0 g, 0.125 mol) was added to a solution of DMAIA (5.0 g, 0.0174 mol, 87 % purity) in methanol (15ml), methylene chloride (70 ml) and tetra-n-butylammonium bromide (0.32 g, 0.1 mol %). The reaction was slightly exothermic and for a quarter of an hour the temperature reached 30 °C. Then the solution was heated at 35 °C for 3 hours. The aqueous phase was separated by funnel and the solvents from the organic phase were distillated. The residue was suspended in water (50 ml) and the precipitate was collected by filtration, washed with water and dried at 50 °C. The yield of crude Moxonidine was 3.56 g (97.58%; toward 87% of DMAIA purity).

### Examples 19-21

### Preparation of further 4-chloro-N-(imidazolidin-2-ylidene)-6-alkoxy-2-methyl-pyrimidin-5-amines by using different alcohols like ethyl alcohol, 2-propanol, n-butyl alcohol

The experiments were made following the conditions in example 2. Further details and the gained results are depicted in table 5 below:

**Table 5- Preparation of further 4-chloro-N-(imidazolidin-2-ylidene)-6-alcoxy-2-methylpyrimidin-5-amines by using different alcohols**

| No | DMAIA [g] ([mol]) | solv. ([ml]) | NaOH [g/ml], % H₂O | T [°C] | t [h] | crude yield [g] ([%]) |
|---|---|---|---|---|---|---|
| 19 | 4.35 (0.0151 ) | EtOH (20) CH₂Cl₂ (100) | 5/10, 50 | 30 | 2.0 | 3.68 (95.35) |
| 20 | 4.35 (0.0151 ) | 2-PrOH (20) CH₂Cl₂ (100) | 5/10, 50 | 30 | 3.5 | 3.86 (94.80) |
| 21 | 4.35 (0.0151 ) | n-BuOH (20) CH₂Cl₂ (100) | 5/10, 50 | 30 | 3.0 | 4.01 (93.65) |

### Legend for tables 1-5 is as follows:

- T: = temperature
- t: = time
- solv.: = solvents
- MeOH: = methanol
- EtOH: = ethanol
- n-BuOH: = n-butanol
- 2-PrOH: = 2-propanol
- CH₂Cl₂: = Methylene chloride
- TBABr: = tetra-n-butylammonium bromide
- TBACJ: = tetra-n-butylammonium iodide
- TEBABr: = triethyl-benzyl ammonium bromide
- TEBACl: = triethyl-benzylammonium chloride
- MTOACl: = methyl-trioctylammonium chloride
- TBAHS: = tetra-n-butylammonium hydrosulfate
- IV: = 4,6-dichloro-2-methyl-5-(2-imidazolin-2-yl)-aminopyrimidine
- V: = 4,6-dimethoxy-2-methyl-5-(2-imidazolin-2-yl)-aminopyrimidine

Besides, the term "crude yield [g] ([%])" in tables 1 to 4 defines the crude yield of Moxonidine in g and %, respectively, while the term "crude yield [g] ([%])" in table 5 defines the crude yield of the particular 4-chloro-N-(imidazolidin-2-ylidene)-6-alkoxy-2-methylpyrimidin-5-amines in g and %, respectively.

Further, the term "purity [%] in tables 1 to 4 defines purity of Moxonidine.

Furthermore, the term "IV [%]" in tables 1 to 4 defines the yield of 4,6-dimethoxy-2-methyl-5-(2-imidazolin-2-yl)-aminopyrimidine (see formula IV), while the term "V [%]" in tables 1 to 4, defines the yield of 4,6-dichloro-2-methyl-5-(2-imidazolin-2-yl)-aminopyrimidine (see formula V).

In summary, the method according to the invention furnished the desired product predominantly in a higher yield and in all cases in a higher purity.

## Claims

1. A method for preparing a compound having the formula (I): 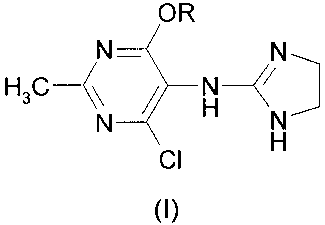
where R is an aliphatic and/or aromatic radical,
**characterized in that** 4,6-dichloro-2-methyl-5-(1-acetyl-2-imidazo-lin-2-yl)-aminopyrimidine and an alcohol are caused to react in the presence of a phase transfer catalyst and that the alcohol is used in combination with at least a further organic solvent.

2. The method according to claim 1, **characterized in that** the alcohol is used in combination with at least one water-insoluble organic solvent.

3. The method according to claim 1 or 2, **characterized in that** the at least one further organic solvent is selected from the group consisting of methylene chloride, chloroform, benzene, diethyl ether, diisopropyl ether, petroleum ether and mixtures thereof.

4. The method according to any of the preceding claims, **characterized in that** the alcohol is selected from the group consisting of methanol, ethanol, n-propanol, 2-propanol, n-butanol, tert. butanol, n-pentanol, n-hexanol, cyclohexanol, allyl alcohol, 3-buten-1-ol, 2-propyn-1-ol, 3-butyn-1-ol, 2-butyn-1-ol, 4-pentyn-1-ol and combinations thereof.

5. Method according to any of the preceding claims, **characterized in that** the compound is Moxonidine (4-chloro-N-(imidazolidin-2-ylidene)-6-methoxy-2-methylpyrimidin-5-amine) and the alcohol is methanol which is caused to react with 4,6-dichloro-2-methyl-5-(1-acetyl-2-imidazolin-2-yl)-aminopyrimidine.

6. The method according to any of the preceding claims, **characterized in that** the phase transfer catalyst is selected from the group consisting of quaternary ammonium salt, in particular alkylammonium salt and/or alkylarylammonium salt, phosphonium salt, crown ether, aza crown ether, cryptand, acyclic molecule being analogous to crown ether, for example oligoethylene glycol and/or polyethylene glycol, and mixtures thereof.

7. The method according to any of the preceding claims, **characterized in that** the phase transfer catalyst is used in an amount of from 0.05 mol % to 5.0 mol %, in particular 0.3 mol % to 3.0 mol %, preferably 0.1 mol %, per one mole 4,6-dichloro-2-methyl-5-(1-acetyl-2-imidazolin-2-yl)-aminopyrimidine.

8. The method according to any of the preceding claims, **characterized in that** the 4,6-dichloro-2-methyl-5-(1-acetyl-2-imidazolin-2-yl)-aminopyrimidine is dissolved in a solvent mixture of the alcohol and preferably at least one further organic solvent being not soluble in water to yield an organic solution.

9. The method according to claim 8, **characterized in that** the phase transfer catalyst is added to the organic solution.

10. The method according to any of the preceding claims, **characterized in that** the reaction is further caused in the presence of an inorganic base.

11. The method according to claim 10, **characterized in that** the inorganic base is a metal hydroxide, in particular selected from the group consisting of alkali metal hydroxide, alkaline earth metal hydroxide and mixtures thereof, more preferably selected from the group consisting of lithium hydroxide, sodium hydroxide, potassium hydroxide, cesium hydroxide, barium hydroxide, magnesium hydroxide, calcium hydroxide, strontium hydroxide and mixtures thereof.

12. The method according to claim 10 or 11, **characterized in that** the inorganic base is provided in form of an aqueous solution.

13. The method according to claim 12, **characterized in that** the aqueous solution contains the inorganic base in an amount of from 10 % by weight to 52 % by weight, in particular 20 % by weight to 51 % by weight, preferably 50 % by weight, related to the total weight of the aqueous solution.

14. The method according to claim 12 or 13, **characterized in that** the aqueous solution is added to the organic solution according to claim 8, wherein the organic solution preferably contains the phase transfer catalyst, thereby forming an organic phase and an aqueous phase.

15. The method according to any of the preceding claims comprising the following steps:
a. providing an organic solution containing the 4,6-dichloro-2-methyl-5-(1-acetyl-2-imidazolin-2-yl)-aminopyrimidine, the alcohol and at least one further organic solvent being insoluble in water,
b. adding a phase transfer catalyst to the organic solution,
c. adding an aqueous solution containing an inorganic base, preferably a metal hydroxide, to the organic solution including the phase transfer catalyst, thereby forming a two-phase system of an organic phase and an aqueous phase,
d. optionally heating the two-phase system,
e. separating the organic phase and aqueous phase,
f. removing, in particular evaporating, the organic solvents from the organic phase, thereby yielding a residue of crude product,
g. suspending the residue, typically in water or in an aqueous liquid, resulting in a precipitate,
h. collecting the precipitate by means of filtration,
i. optionally washing the collected precipitate, preferably with water or an aqueous liquid and
j. drying the washed precipitate, thereby yielding purified and in particular crystalline compound having the formula (I).

## Patentansprüche

1. Verfahren zur Herstellung einer Verbindung mit der Formel (I): 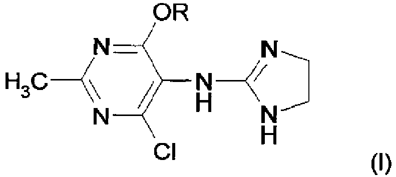
worin R ein aliphatischer und/oder aromatischer Rest ist,
**dadurch gekennzeichnet, dass** 4,6-Dichlor-2-methyl-5-(1-acetyl-2-imidazolin-2-yl)-aminopyrimidin und ein Alkohol in Gegenwart eines Phasentransferkatalysators zur Reaktion gebracht werden, und dass der Alkohol in Kombination mit mindestens einem weiteren organischen Lösungsmittel verwendet wird.

2. Verfahren nach Anspruch 1, **dadurch gekennzeichnet, dass** der Alkohol in Kombination mit mindestens einem wasserunlöslichen organischen Lösungsmittel verwendet wird.

3. Verfahren nach Anspruch 1 oder 2, **dadurch gekennzeichnet, dass** das mindestens eine weitere organische Lösungsmittel ausgewählt ist aus der Gruppe bestehend aus Methylenchlorid, Chloroform, Benzol, Diethylether, Diisopropylether, Petrolether und Mischungen davon.

4. Verfahren nach einem der vorhergehenden Ansprüche, **dadurch gekennzeichnet, dass** der Alkohol ausgewählt ist aus der Gruppe bestehend aus Methanol, Ethanol, n-Propanol, 2-Propanol, n-Butanol, tert-Butanol, n-Pentanol, n-Hexanof, Cyclohexanol, Allylalkohol, 3-Buten-1-ol, 2-Propin-1-ol, 3-Butin-1-ol, 2-Butin-1-ol, 4-Pentin-1-ol und Kombinationen davon.

5. Verfahren nach einem der vorhergehenden Ansprüche, **dadurch gekennzeichnet, dass** die Verbindung Moxonidin (4-Chlor-N-(imidazolidin-2-yliden)-6-methoxy-2-methylpyrimidin-5-amin) ist und der Alkohol Methanol ist, der mit 4,6-Dichlor-2-methyl-5-(1-acetyl-2-imidazolin-2-yl)-aminopyrimidin zur Reaktion gebracht wird.

6. Verfahren nach einem der vorhergehenden Ansprüche, **dadurch gekennzeichnet, dass** der Phasentransferkatalysator ausgewählt ist aus der Gruppe bestehend aus einem quaternären Ammoniumsalz, insbesondere Alkylammoniumsalz und/oder Alkylarylammoniumsalz, Phosphoniumsalz, Kronenether, Aza-Kronenether, Cryptand, zum Kronenether analoges acyclisches Molekül, beispielsweise Oligoethylenglykol und/oder Polyethylenglykol und Mischungen davon.

7. Verfahren nach einem der vorhergehenden Ansprüche, **dadurch gekennzeichnet, dass** der Phasentransferkatalysator in einer Menge von 0,05 Mol-% bis 5,0 Mol-%, insbesondere 0,3 Mol-% bis 3,0 Mol-%, bevorzugt 0,1 Mol-% pro Mol 4,6-Dichlor-2-methyl-5-(1-acetyl-2-imidazolin-2-yl)-aminopyrimidin eingesetzt wird.

8. Verfahren nach einem der vorhergehenden Ansprüche, **dadurch gekennzeichnet, dass** das 4,6-Dichlor-2-methyl-5-(1-acetyl-2-imidazolin-2-yl)-aminopyrimidin in einem Lösungsmittelgemisch aus dem Alkohol und bevorzugt mindestens einem weiteren organischen Lösungsmittel, das nicht in Wasser löslich ist, gelöst wird, so dass sich eine organische Lösung ergibt.

9. Verfahren nach Anspruch 8, **dadurch gekennzeichnet, dass** der Phasentransferkatalysator der organischen Lösung zugegeben wird.

10. Verfahren nach einem der vorhergehenden Ansprüche, **dadurch gekennzeichnet, dass** die Reaktion weiter in Gegenwart einer anorganischen Base erfolgt.

11. Verfahren nach Anspruch 10, **dadurch gekennzeichnet, dass** die anorganische Base ein Metallhydroxid ist, insbesondere ausgewählt aus der Gruppe bestehend aus Alkalimetallhydroxid, Erdalkalimetallhydroxid und Mischungen davon, bevorzugt ausgewählt aus der Gruppe bestehend aus Lithiumhydroxid, Natriumhydroxid, Kaliumhydroxid, Caesiumhydroxid, Bariumhydroxid, Magnesiumhydroxid, Calciumhydroxid, Strontiumhydroxid und Mischungen davon.

12. Verfahren nach Anspruch 10 oder 11, **dadurch gekennzeichnet, dass** die anorganische Base in Form einer wässrigen Lösung bereitgestellt wird.

13. Verfahren nach Anspruch 12, **dadurch gekennzeichnet, dass** die wässrige Lösung die anorganische Base in einer Menge von 10 Gew.-% bis 52 Gew.-%, insbesondere 20 Gew.-% bis 51 Gew.-%, bevorzugt 50 Gew.-%, bezogen auf das Gesamtgewicht der wässrigen Lösung, enthält.

14. Verfahren nach Anspruch 12 oder 13, **dadurch gekennzeichnet, dass** die wässrige Lösung der organischen Lösung nach Anspruch 8 zugegeben wird, wobei die organische Lösung bevorzugt den Phasentransferkatalysator enthält, wodurch eine organische Phase sowie eine wässrige Phase ausgebildet werden.

15. Verfahren nach einem der vorhergehenden Ansprüche, umfassend die folgenden Schritte:
a. Bereitstellen einer organischen Lösung, die 4,6-Dichlor-2-methyl-5-(1-acetyl-2-imidazolin-2-yl)-aminopyrimidin, den Alkohol und mindestens ein weiteres organisches Lösungsmittel, das in Wasser unlöslich ist, enthält,
b. Zugeben eines Phasentransferkatalysators zu der organischen Lösung,
c. Zugeben einer wässrigen Lösung, die eine anorganische Base, bevorzugt ein Metallhydroxid, enthält, zur organischen Lösung mit dem Phasentransferkatalysator, wodurch ein Zweiphasensystem aus einer organischen Phase und einer wässrigen Phase gebildet wird,
d. optional Erwärmen des Zweiphasensystems,
e. Trennen der organischen Phase und der wässrigen Phase,
f. Entfernen, insbesondere Verdampfen, der organischen Lösungsmittel aus der organischen Phase, wodurch ein Rückstand von Rohprodukt erhalten wird,
g. Suspendieren des Rückstands, typischerweise in Wasser oder in einer wässrigen Flüssigkeit, so dass sich ein Niederschlag bildet,
h. Aufnehmen des Niederschlags durch Filtration,
i. optional Waschen des aufgenommenen Niederschlags, bevorzugt mit Wasser oder einer wässrigen Flüssigkeit und
j. Trocknen des gewaschenen Niederschlags, wodurch eine gereinigte und insbesondere kristalline Verbindung mit der Formel (I) erhalten wird.

## Revendications

1. Procédé de préparation d'un composé de formule (I) : 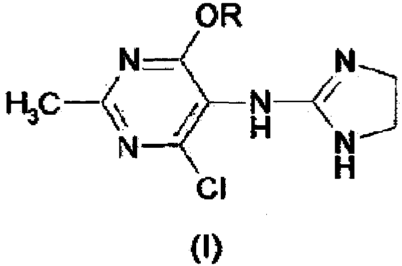
dans lequel R est un radical aliphatique et/ou aromatique,
**caractérisé en ce qu'**on fait réagir la 4,6-dichloro-2-méthyl-5-(1-acétyl-2-imidazolin-2-yl)aminopyrimidine et un alcool en présence d'un catalyseur de transfert de phase, et **en ce que** l'alcool est utilisé en association avec au moins un autre solvant organique.

2. Procédé selon la revendication 1, **caractérisé en ce que** l'alcool est utilisé en association avec au moins un solvant organique insoluble dans l'eau.

3. Procédé selon la revendication 1 ou 2, **caractérisé en ce que** l'au moins un autre solvant organique est choisi dans le groupe constitué du chlorure de méthylène, du chloroforme, du benzène, du diéthyléther, du diisopropyléther, de l'éther de pétrole et de leurs mélanges.

4. Procédé selon l'une quelconque des revendications précédentes, **caractérisé en ce que** l'alcool est choisi dans le groupe constitué du méthanol, de l'éthanol, du n-propanol, du propan-2-ol, du n-butanol, du tertiobutanol, du n-pentanol, du n-hexanol, du cyclohexanol, de l'alcool allylique, du but-3-én-1-ol, prop-2-yn-1-ol, du but-3-yn-1-ol, du but-2-yn-1-ol, du pent-4-yn-1-ol, et de leurs mélanges.

5. Procédé selon l'une quelconque des revendications précédentes, **caractérisé en ce que** le composé est de la moxonidine (4-chloro-N-(imidazolidin-2-ylidène)-6-méthoxy-2-méthylpyrimidin-5-amine) et l'alcool est du méthanol, qu'on fait réagir avec la 4,6-dichloro-2-méthyl-5-(1-acétyl-2-imidazolin-2-yl)aminopyrimidine.

6. Procédé selon l'une quelconque des revendications précédentes, **caractérisé en ce que** le catalyseur de transfert de phase est choisi dans le groupe constitué d'un sel d'ammonium quaternaire, en particulier d'un sel d'alkylammonium et/ou d'un sel d'alkylarylammonium, d'un sel de phosphonium, d'un éther couronne, d'un éther aza-couronne, d'un cryptand, d'une molécule acyclique analogue d'un éther couronne, par exemple un oligoéthylène glycol et/ou le polyéthylène glycol, et leurs mélanges.

7. Procédé selon l'une quelconque des revendications précédentes, **caractérisé en ce que** le catalyseur de transfert de phase est utilisé à hauteur de 0,05 % en moles à 5,0 % en moles, en particulier de 0,3 % en moles à 3,0 % en moles, de préférence de 0,1 % en moles par mole de 4,6-dichloro-2-méthyl-5-(1-acétyl-2-imidazolin-2-yl)aminopyrimidine.

8. Procédé selon l'une quelconque des revendications précédentes, **caractérisé en ce que** la 4,6-dichloro-2-méthyl-5-(1-acétyl-2-imidazolin-2-yl)aminopyrimidine est dissoute dans un mélange solvant de l'alcool et de préférence d'un autre solvant organique non soluble dans l'eau, pour donner une solution organique.

9. Procédé selon la revendication 8, **caractérisé en ce que** le catalyseur de transfert de phase est ajouté à la solution organique.

10. Procédé selon l'une quelconque des revendications précédentes, **caractérisé en ce que** la réaction est, en outre, provoquée en présence d'une base minérale.

11. Procédé selon la revendication 10, **caractérisé en ce que** la base minérale est un hydroxyde métallique, choisi en particulier dans le groupe constitué des hydroxydes de métal alcalin, des hydroxydes de métal alcalino-terreux, et de leurs mélanges, plus préférentiellement choisi dans le groupe constitué de l'hydroxyde de lithium, de l'hydroxyde de sodium, de l'hydroxyde de potassium, de l'hydroxyde de césium, de l'hydroxyde de baryum, de l'hydroxyde de magnésium, de l'hydroxyde de calcium, de l'hydroxyde de strontium et de leurs mélanges.

12. Procédé selon la revendication 10 ou 11, **caractérisé en ce que** la base minérale est fournie sous forme d'une solution aqueuse.

13. Procédé selon la revendication 12, **caractérisé en ce que** la solution aqueuse contient la base minérale à hauteur de 10 % en poids à 52 % en poids, en particulier de 20 % en poids à 51 % en poids, préférentiellement de 50 % en poids rapportés au poids total de la solution aqueuse.

14. Procédé selon la revendication 12 ou 13, **caractérisé en ce que** la solution aqueuse est ajoutée à la solution organique selon la revendication 8, la solution organique contenant le catalyseur de transition de phase, formant ainsi une phase organique et une phase aqueuse.

15. Procédé selon l'une quelconque des revendications précédentes, comprenant les étapes suivantes :
a. production d'une solution organique contenant la 4,6-dichloro-2-méthyl-5-(1-acétyl-2-imidazolin-2-yl)aminopyrimidine, l'alcool et au moins un autre solvant organique insoluble dans l'eau,
b. ajout d'un catalyseur de transfert de phase à la solution organique,
c. ajout d'une solution aqueuse contenant une base minérale, préférentiellement un hydroxyde de métal, à la solution organique contenant le catalyseur de transfert de phase, formant ainsi un système biphasique d'une phase organique et une phase aqueuse,
d. facultativement, chauffage du système biphasique,
e. séparation de la phase organique et de la phase aqueuse,
f. élimination, en particulier évaporation des solvants organiques de la phase organique, produisant ainsi un résidu de produit brut,
g. mise en suspension du résidu, typiquement dans l'eau ou dans un liquide aqueux, donnant un précipité,
h. collecte du précipité par filtration,
i. facultativement, lavage du précipité collecté, préférentiellement avec de l'eau ou un liquide aqueux, et
j. séchage du précipité lavé, donnant un composé purifié, et en particulier cristallin, de formule (I).
